# EUROPEAN PATENT APPLICATION

(11) **EP 4 450 626 A1**
(43) Date of publication of application: **23.10.2024**
(21) Application number: 23169158.5
(22) Date of filing: 21.04.2023
(51) Int. Cl.: C12N 15/113, A61K 31/7088, A61P 3/00

(54) **FNIP2 INHIBITORS FOR TREATING ATAXIA TELANGIECTASIA**

(71) Applicant: IFOM - Istituto Fondazione di Oncologia Molecolare ETS, 20139 Milano (IT)
(72) Inventor: Vicenzo, Costanzo, Frattamaggiore (NA) (IT)
(74) Representative: Pace Napoleone, Maria

(57) **Abstract**

The present invention relates to inhibitors of the FNIP2 protein, in particular to a polynucleotide able to inhibit at least partially expression of the FNIP2 protein, wherein said polynucleotide is selected from a short hairpin RNA (shRNA), an oligonucleotide expressing or coding for a shRNA and an antisense oligonucleotide.

The invention also relates to inhibitors of the FNIP2 protein for use in the treatment of Ataxia Telangiectasia or of associates diseases.

Vectors and pharmaceutical compositions comprising said inhibitors are also within the scope of the invention.

## Description

### FIELD OF THE INVENTION

The present invention relates to the field of treatments of Ataxia Telangiectasia.

In particular, it is related to inhibitors of FNIP2 for use in the treatment of Ataxia Telangiectasia.

### BACKGROUND OF THE INVENTION

Ataxia Telangiectasia (AT) is an autosomal recessive disease that displays a complex phenotype. Patients exhibit neurological impairments due to progressive cerebellar degeneration, ocular telangiectasia, immunodeficiency, premature aging, radiation sensitivity, pulmonary disease, increased risk for cancer and diabetes (Shiloh, 2014). Ataxia-telangiectasia mutated (ATM) protein kinase can be activated by DNA double strand breaks and by reactive oxygen species (ROS), leading to the phosphorylation of several proteins (Matsuoka et al., 2007). ATM protein loss and malfunction have been implicated in mammalian models of metabolic disease, which is characterized by insulin resistance, increased cholesterol and lipid levels, high blood pressure, and atherosclerosis (Shoelson, 2006). Our previous work has shown that ATM stimulates the pentose phosphate pathway (PPP) (Cosentino et al., 2011), a pathway to metabolize glucose that operates in parallel with glycolysis. PPP is a major source of cellular constituents, including nucleotide precursor ribose-5 phosphate, and NADPH, an essential antioxidant cofactor. ATM upregulates the PPP by increasing the activity of the rate limiting enzyme glucose-6-phospate dehydrogenase (G6PD), which plays a fundamental role in cell growth and signaling, embryonic development and degenerative diseases. ATM dependent stimulation of the PPP might facilitate DNA repair and promote cell survival through increased production of nucleotide pre-cursors and antioxidant molecules. Consistent with these findings, links between ATM and cellular metabolism have increasingly been reported(Ambrose and Gatti, 2013). Overall, these observations indicate a role for ATM in regulating cellular metabolism under normal and challenged conditions.

ATM dependent control of the PPP and the pathways linked to the PPP may be critical for cell survival even in the absence of apparent DNA damage. Consistent with imbalanced redox control in AT cells lower antioxidant levels correlate with the severity of neurological impairment in AT patients and that steroid treatment mediated improvement of clinical symptoms correlates with higher reducing agent production (Russo et al., 2009). Primary cells derived from A-T patients grow poorly and undergo premature senescence (Shiloh et al., 1982).

However how unresolved ROS induced cellular stress impact on general AT cell growth, proliferation is poorly understood.

Therefore, there is still the need of a treatment to improve AT cellular defects and consequently the symptoms of Ataxia Telangiectasia.

Inventors performed extensive metabolomics, flux and histological analyses on normal and human AT cells and tissues that revealed a striking accumulation of glycogen, the major storage polymer of glucose. They showed that glycogen accumulation result from ineffective glycolysis (Hicks et al., 2011). Glycogen accumulation might contribute to explain glucose metabolism imbalances leading to insulin resistance, diabetes and heart failure (Schalch et al., 1970; Wingard et al., 2020) in AT patients. Strikingly, accumulation of glycogen was observed also in human neurons and brains, where it might contribute to A-T neuropathology. To understand whether defective glycolysis linked to glycogen accumulation was responsible to AT cellular defects inventors looked for strategies to rescue glucose metabolism.

### SUMMARY OF THE INVENTION

It was now found that ineffective glycolysis can be overcome by suppression of FNIP2, member of the FNIP2-FNIP1-Folliculin (FFF) complex, which negatively regulates energy metabolism. Suppression of FNIP2 restored normal glucose metabolism and mitochondrial respiration in A-T cells promoting normal cell growth and cell proliferation of AT primary cells. These results suggest the existence of a FNIP2-dependent metabolic checkpoint that restrains glucose metabolism and cell growth in the absence of ATM. These findings can have an immediate impact for AT at therapeutic levels. Also, LNA oligos able to downregulate FNIP2 mRNA levels were found which improved survival of AT fibroblasts and iPSC-derived neurons.

It is an object of the invention a polynucleotide able to inhibit at least partially expression of the FNIP2 protein, wherein said polynucleotide is selected from a short hairpin RNA (shRNA), an oligonucleotide expressing or coding for a shRNA and an antisense oligonucleotide.

A further object of the invention is a pharmaceutical composition comprising said polynucleotide and at least one pharmaceutically accepted vehicle and/or excipient.

A further object of the invention is a vector comprising or expressing said polynucleotide.

It is also an object of the invention an inhibitor of protein FNIP2 for use in the treatment of Ataxia Telangiectasia.

In particular, said inhibitor is for use for the treatment and/or prevention of cancer and/or of a neurological disease or impairment in a subject affected by Ataxia Telangiectasia.

In some embodiments, said inhibitor of the FNIP2 protein can be selected from:
a) a polynucleotide;
b) a vector comprising or expressing said polynucleotide;
c) an host cell genetically engineered to express said polynucleotide;
d) a small molecule;
e) a peptide, a protein, an antibody, an antisense oligonucleotide, a siRNA, a shRNA, a locked nucleic acid (LNA) oligonucleotide, a recombinant virus, an antisense expression vector.

In a preferred embodiment, said inhibitor is a polynucleotide, preferably selected from a short hairpin RNA (shRNA) and an antisense oligonucleotide, preferably a LNA antisense oligonucleotide.

A further object of the invention is a pharmaceutical composition comprising at least one inhibitor of FNIP2 protein and at least one pharmaceutically accepted vehicle and/or excipient for use in the treatment of Ataxia Telangiectasia.

A further object of the invention is a vector comprising or expressing an inhibitor of the FNIP2 protein, in particular an inhibitor of the expression of the FNIP2 gene, for use in the treatment of Ataxia Telangiectasia.

### DETAILED DESCRIPTION OF THE INVENTION

### Figures

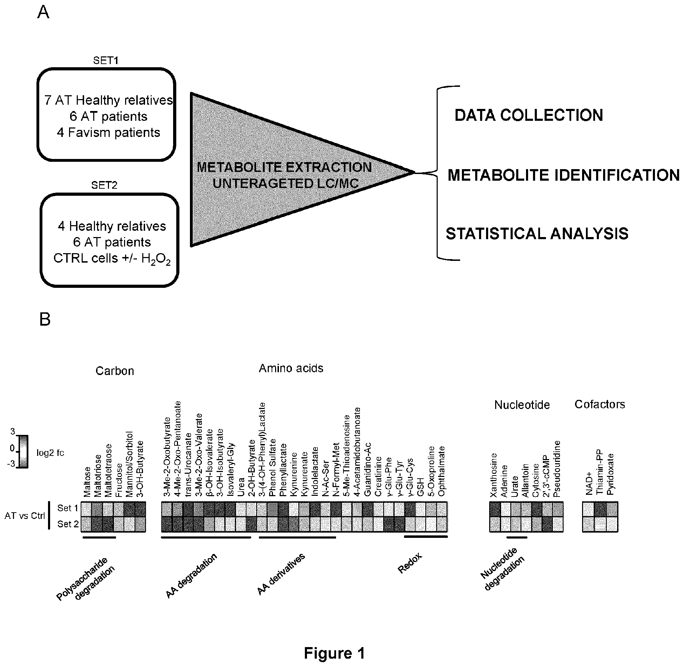

**Figure 1****: Carbon source degradation and oxidative stress signature in primary AT cells.** A) Schematics of metabolite analysis on two sets of samples in independent experiments. Metabolite identification and relative levels were established through LC-MS and GS-MS of 6 replicates collected for each condition. Statistical assessment was performed with R package maanova using an FDR threshold of 0.1 for the identification of significant metabolite alterations. B) Heat maps showing relative levels of significant metabolites in the two sets for the indicated super-pathways (carbon, amino acid, nucleotide and cofactor metabolism). Selected metabolic processes are indicated. Not detected metabolites are shown as grey boxes. Lipid metabolites are not shown.

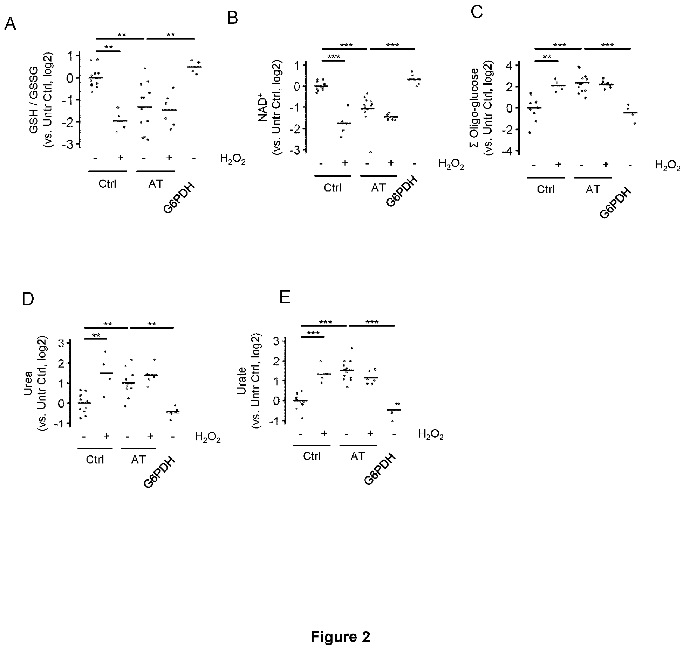

**Figure 2****: Oxidative stress as major determinant of metabolic alterations in AT cells.** Graphs showing A) GSH/GSSG ratio (*p*_{ANOVA}: 1.4 x 10⁻⁵; *p*_{adj} Ctrl + H₂O₂ vs. Ctrl - H₂O₂: 0.0013; *p*_{adj} Ctrl - H₂O₂ vs. AT - H₂O₂: 0.0019; *p*_{adj} AT - H₂O₂ vs. G6PDH: 0.0022), B) NAD⁺ (*p*_{ANOVA}: 2.8 x 10⁻⁸; *p*_{adj} Ctrl + H₂O₂ vs. Ctrl - H₂O₂: 7.5 x 10⁻⁶; *p*_{adj} Ctrl - H₂O₂ vs. AT - H₂O₂: 9.8 x 10⁻⁵; *p*_{adj} AT - H₂O₂ vs. G6PDH: 2.3 x 10⁻⁴), C) Σ-Oligosaccarides (*p*_{ANOVA}: 1.3 x 10⁻⁷; *p*_{adj} Ctrl + H₂O₂ vs. Ctrl - H₂O₂: 0.002; *p*_{adj} Ctrl - H₂O₂ vs. AT - H₂O₂: 2.6 x 10⁻⁶; *p*_{adj} AT - H₂O₂ vs. G6PDH: 3.2 x 10⁻⁵), D) Urea (*p*_{ANOVA}: 2.6 x 10⁻⁶; *p*_{adj} Ctrl + H₂O₂ vs. Ctrl - H₂O₂: 9.7 x 10⁻⁴; *p*_{adj} Ctrl - H₂O₂ vs. AT - H₂O₂: 0.0019; *p*_{adj} AT - H₂O₂ vs. G6PDH: 0.0011) and E) Urate (*p*_{ANOVA}: 5.6 x 10⁻¹⁰; *p*_{adj} Ctrl + H₂O₂ vs. Ctrl - H₂O₂: 1.1 x 10⁻⁴; *p*_{adj} Ctrl - H₂O₂ vs. AT - H₂O₂: 2.1 × 10⁻⁸; *p*_{adj} AT - H₂O₂ vs. G6PDH: 5.9 x 10⁻⁸) levels in CTRL and AT cells untreated or exposed to H₂O₂ and in untreated G6PDH mutant cells compared to untreated CTRL cells. Untreated Ctrl and AT cells were used from both sample sets, untreated G6PDH cells from set 1 and H2O2-treated cells from set 2 (see Figure 1A). Significance in A-E was calculated by 1-way ANOVA with post-hoc Tukey HSD test.

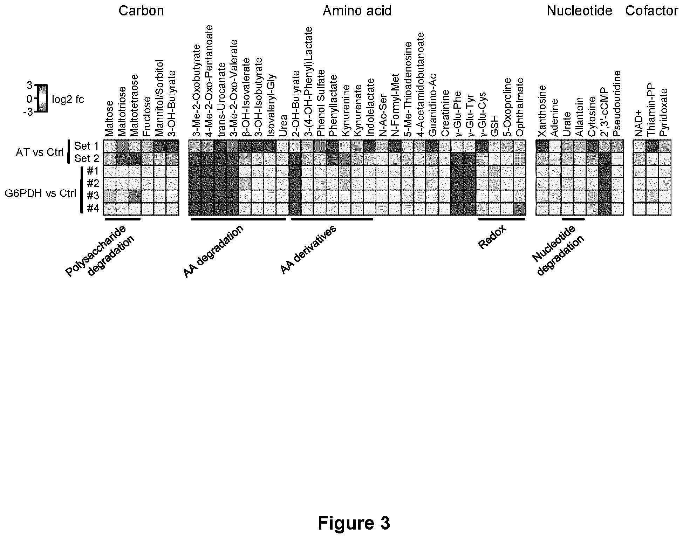

**Figure 3****. Metabolites in AT versus G6PD deficient cells.** Heat maps showing relative levels of significant metabolites in the indicated sets for the indicated super-pathways (carbon, amino acid, nucleotide and cofactor metabolism). Selected metabolic processes are indicated. Not detected metabolites are shown as grey boxes. Lipid metabolites are not shown.

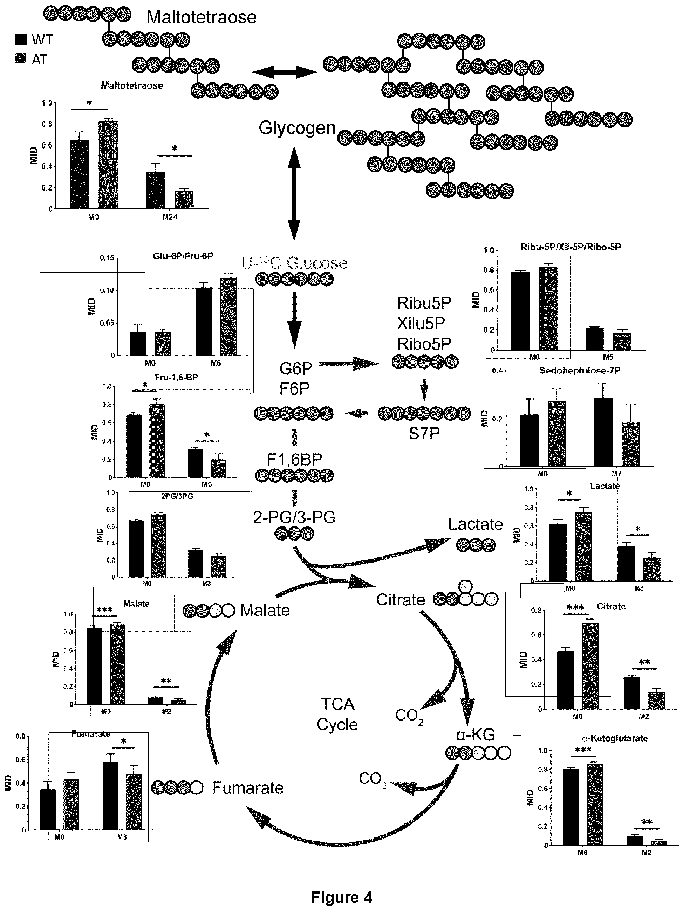

**Figure 4****: Flux analysis showing major glucose metabolism related alterations in AT cells.** MID Mass isotopomer distributions (MID) for each intermediate indicated in the graph in CTRL and AT cells. U-¹³C position is indicated as filled circle. Statistical analysis was conducted using unpaired t-test. *P<0,05; **P<0,01; ***P<0,001. N=5.

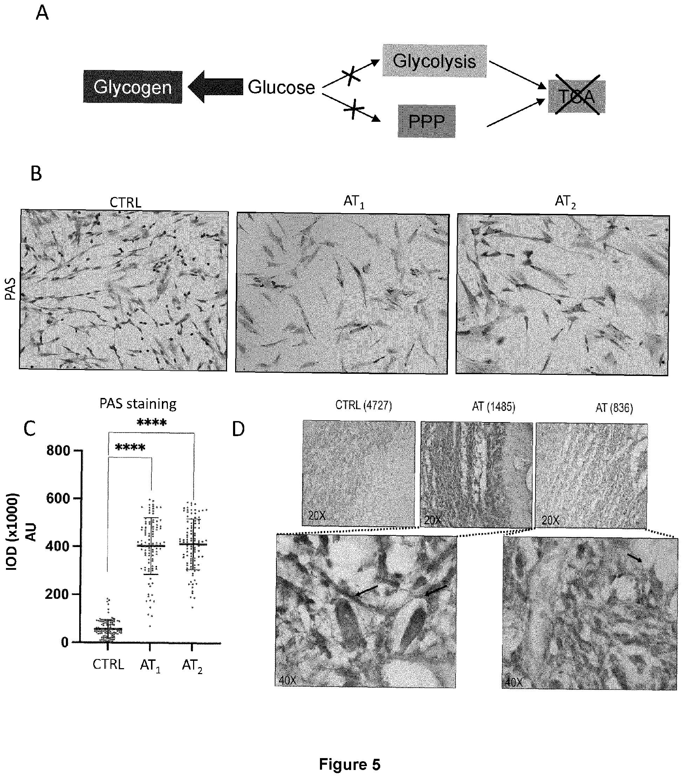

**Figure 5****. Glycogen accumulation in AT cells and tissues.** A) Schematics showing the effects of inefficient glycolysis, PPP and TCA on glucose and glycogen levels. B) PAS staining of CTRL and AT primary fibroblast derived from two patients (AT₁ and AT₂). C) Integrate optical density for PAS staining of the samples shown in A. Each dot represents a single cell. D) Immunostaining of cerebellar sections from CTRL subject and two different AT patients using an anti-glycogen antibody. Arrows in zoomed windows indicate Purkinje cells.

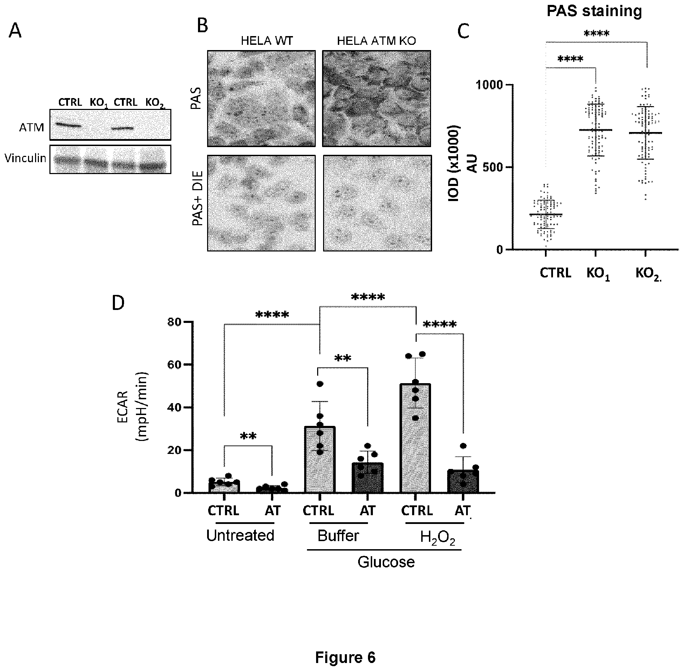

**Figure 6****. ATM induces glycolysis and glycogen clearing.** A) WB showing CRISPR/Cas9 mediated ATM gene knock out HeLa cells. B) PAS staining of CTRL and HeLa ATM KO cells before and after disterase (Die). C) Integrated optical density for PAS staining of the samples shown in B. Each dot represents a single cell. Statistical analysis was conducted using multiple upaired t-test. ****P<0,001. 100 cells pooled from independent experiments were scored for each sample (n=100). D) Extracellular acidification (ECAR) in CTRL and AT cells in untreated and following supplementation of 30 mM glucose in the presence of buffer or 50 mM H₂O₂.

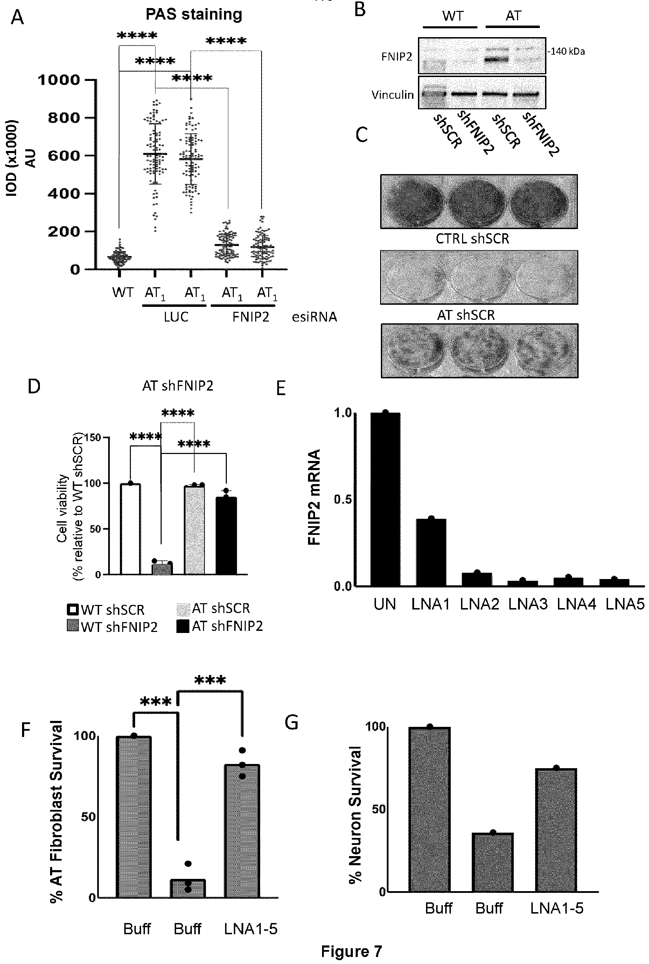

**Figure 7****. Inactivation of FNIP2 suppresses glycogen accumulation promoting AT cell survival.** A) Integrated optical density for PAS staining of the samples treated as indicated in the graph legend. Each dot represents a single cell. Statistical analysis was conducted using multiple upaired t-test. ****P<0,001. 100 cells pooled from independent experiments were scored for each sample (n=100). B) WB of the indicated proteins in CTRL and AT cells infected with shSCR or shFNIP2. C) Colony assay stained with crystal violet of CTRL and AT cells infected with shSCR or shFNIP2 as indicated. D) Cell viability relative to CTRL cells of CTRL and AT cells infected with shSCR or shFNIP2. Statistical analysis was conducted using multiple unpaired t-test. ****P<0,001. Colonies for each sample from three independent experiments were scored (n=3). E) Relative levels of FNIP2 mRNA measured by real time PCR in fibroblasts that were untreated (UN) or treated with five different LNA oligos (LNAs 1 to 5) as indicated. F) Graph showing relative survival of AT compared control (CTRL) fibroblasts cultivated in the presence of buffer or a mixture of LNA1-5 oligos. G) Graph showing relative survival of AT compared control (CTRL) iPSCs-derived neurons cultivated in the presence of buffer or a mixture of LNA1-5 oligos.

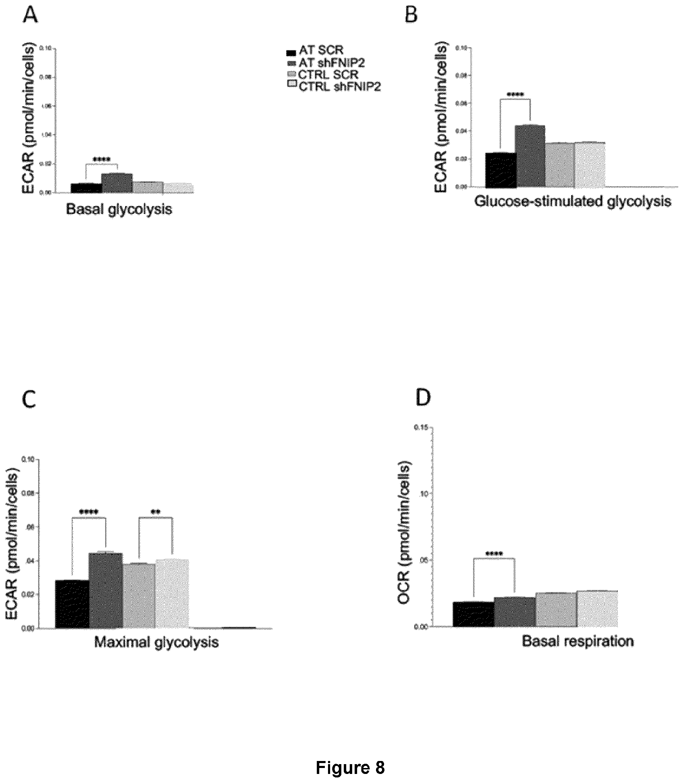

**Figure 8****. Inactivation of FNIP2 reactivates glycolysis in AT cells promoting.** A) Quantification of basal glycolysis levels in CTRL and AT cells treated infected with shSCR or shFNIP2 as indicated. B) Quantification of glucose stimulated glycolysis levels in samples indicated in A. C) Quantification of maximal glycolysis levels in samples indicated in A. D) Quantification of oxygen consumption rate (OCR) in samples shown in A. Gray scale legend shown in A applies also to graphs in B, C and D.

In the context of the present invention, for Ataxia Telangiectasia (AT or A-T) is intended a neurodegenerative, autosomal recessive disease caused by a defect in the ATM gene. Ataxia-telangiectasia syndrome or Louis-Bar syndrome are synonyms.

In the context of the present invention, for ATM it is intended the ataxia-telangiectasia mutated protein, a serine/threonine protein kinase, or the corresponding gene.

In the context of the present invention, for FNIP2 protein or FNIP2 it is intended the Folliculin Interacting Protein 2, a member of the FNIP2-FNIP1-Folliculin (FFF) complex. Any variant or form of this protein is included in the term, in particular the human variant. For FNIP2 gene it is intended the FNIP2 gene coding for the FNIP2 protein. Reference can be made for example to the gene with NCBI Entrez Gene ID 57600 (database version of 29 March 2023) and to the protein with Uniprot ID Q9P278 (database version of 22 February 2023).

In the context of the present invention, for inhibitor of the FNIP2 protein is intended an agent able to decrease or block, at least partially, the activity of the FNIP2 protein. It is also intended an agent able to decrease, silence or block, at least partially, the expression of the gene coding for the FNIP2 protein.

In the context of the present invention, for "inhibit expression of the FNIP2 protein" it is intended that the agent decreases, silences or blocks, at least partially, the expression of the gene coding for the FNIP2 protein.

In the context of the present invention, for polynucleotide is intended a polymer of nucleotides, which can be ribonucleotides or deoxyribonucleotides, of any length. Therefore, the term polynucleotide includes DNA or RNA, single or double strand, genomic DNA, cDNA, DNA-RNA hybrid or a polymer comprising natural, chemically or biochemically modified, non-natural or functionalized nucleotide bases. In particular, the polynucleotide may also comprise LNAs. The term "nucleic acid" is herein intended as a synonym.

In the context of the present invention, the terms "peptide", "polypeptide" or "protein" are used interchangeably and refer to a polymer of amino acids of any length, which may comprise natural or chemically or biochemically modified or non-natural or functionalized amino acids.

In the context of the present invention, for short hairpin RNA or shRNA is intended an artificial RNA molecule with a tight hairpin turn that can be used to silence target gene expression via RNA interference (RNAi). Small hairpin RNA is a synonym.

Within the context of the present invention, the term "locked nucleic acid" or "LNA" means a nucleotide with the ribose ring blocked in an N-type conformation by a 2'-O,4'-C bridge. LNAs are described for example in WO 99/14226, WO 00/56746, WO 00/56748, WO 01/25248, WO 02/28875, WO 03/006475 and WO 03/095467 and the references mentioned therein.

According to the present invention, it is an object of the invention a polynucleotide able to inhibit expression of FNIP2 protein, wherein said polynucleotide is selected from a short hairpin RNA (shRNA), an oligonucleotide expressing or coding for a shRNA and an antisense oligonucleotide. In particular, said polynucleotide is able to bind the gene coding for the FNIP2 protein and decrease or block, at least partially, its expression.

It is well known in the field how to identify a polynucleotide able to inhibit the expression of a known gene. In particular, shRNA and LNA antisense oligonucleotides are commonly used to inhibit the activity of target genes. A skilled person in the field can design or purchase shRNA or LNA antisense oligonucleotides able to inhibit the expression of the FNIP2 gene.

In a preferred embodiment, said FNIP2 inhibitor is an oligonucleotide comprising from 10 to 40 nucleotides. Preferably, said polynucleotide or said oligonucleotide is able to bind at least partially to the gene coding for FNIP2 and consequently inhibit, at least partially, its expression or said polynucleotide or said oligonucleotide codifies for or expresses a molecule able to bind the gene coding for FNIP2 and inhibit at least partially its expression.

In an embodiment, said polynucleotide is a shRNA. Said shRNA preferably comprises a sequence complementary, at least in part, to the sequence of the gene coding for the FNIP2 protein. Said shRNA is preferably delivered by an expression vector.

In an embodiment, said polynucleotide is an oligonucleotide expressing or coding for a shRNA, said shRNA which preferably comprises a sequence complementary, at least in part, to the sequence of the gene coding for the FNIP2 protein.

For oligonucleotide expressing or coding for a shRNA is intended an oligonucleotide which when included in a vector and delivered to a cell is transcribed to an RNA which is a shRNA.

Said oligonucleotide expressing a shRNA can be included in a vector, such as a plasmid or a viral or bacterial vector. When the oligonucleotide is delivered to the cells the shRNA is expressed and can bind the target gene.

In another embodiment, said polynucleotide is an antisense oligonucleotide. For antisense oligonucleotide it is intended an oligonucleotide comprising at least 5 nucleotides capable of binding to the FNIP2 gene by complementary base pairing, thereby decreasing or blocking the expression of said gene. For "complementary base pairing" it is intended that some or all of the bases of said oligonucleotide pair with some or all of the bases of said gene. In particular, said inhibitor can be able to pair at least 40% of its bases with the bases of the target gene; for example said inhibitor is able to pair at least 40%, 50%, 60%, 70%, 80%, 90% or 100% of its bases with the bases of the target gene. The term "base" means a nucleobase, i.e. the nitrogen-containing portion of nucleosides, which in turn are components of nucleotides.

In an embodiment, said oligonucleotide comprises one or more phosphorothioathe bonds between any of the nucleotides, preferably within all the nucleotides.

In an embodiment, said antisense oligonucleotide comprises at least one modified nucleotide, preferably selected from a locked nucleic acid (LNA)-modified nucleotide, a 2'-O-methyl-modified nucleotide, a 2'-O-methoxyethyl modified nucleotide and a 20-[2-(N-Methylcarbamoyl)ethyl]- modified nucleotide.

In a particular embodiment, said oligonucleotide comprises at least one locked nucleic acid (LNA) and it is herein named also "LNA oligonucleotide" or "LNA antisense oligonucleotide". The LNA oligonucleotide may also comprise one or more phosphorothioathe bonds between the nucleotides.

In a preferred embodiment, said oligonucleotide comprises at least 8 LNAs, preferably between 8 and 10 LNAs. More preferably, said oligonucleotide comprises at least one LNA at the beginning of the oligonucleotide and at least one LNA at the end of the oligonucleotide. In a preferred embodiment, said oligonucleotide comprises 4 to 6 LNAs on each end of the oligonucleotide; this means that said oligonucleotide comprises 4 to 6 contiguous LNAs, preferably 4, at the beginning of the oligonucleotide and 4 to 6 contiguous LNAs, preferably 4, at the end of the oligonucleotide. In this embodiment, the oligonucleotide typically further comprises further non-modified nucleotides between the LNAs and it can be referred to as "gapmer".

In a preferred embodiment, said inhibitor is a LNA antisense oligonucleotide comprising or consisting of a sequence selected from the following sequences:
+A*+G*+T*+A*G*G*T*C*A*G*G*A*+C*+A*+T*+A [SEQ ID N. 1]
+G*+A*+A*+A*G*T*A*G*G*T*C*A*+G*+G*+A*+C [SEQ ID N. 2]
+G*+T*+G*+G*A*G*G*A*G*A*A*C*+G*+T*+A*+T [SEQ ID N. 3]
+A*+C*+G*+G*C*T*G*A*C*A*T*A*+A*+A*+A*+C [SEQ ID N. 4]
+C*+G*+A*+T*T*C*T*G*A*G*G*A*+C*+A*+G*+C [SEQ ID N. 5]
wherein the nucleotides preceded by a symbol "+" are LNA and the symbol "*" indicates a phosphorothioathe bond between the nucleotides.

Therefore, in each of the above mentioned oligonucleotides with SEQ ID N. 1, 2, 3, 4 and 5 the first four nucleotides and the last four nucleotides are LNAs and all nucleotides are bound by phosphorothioathe bonds.

For phosphorothioate bond is intended the substitution with a sulfur atom of a non-bridging oxygen in the phosphate backbone of the oligonucleotide.

In a preferred embodiment, said polynucleotide is a mixture comprising two or more oligonucleotides having the sequences with SEQ ID N. 1, 2, 3, 4 and 5, preferably comprising oligonucleotides having all said sequences.

A vector comprising or expressing said polynucleotide is also within the scope of the invention.

Said vector can be viral or non-viral, for example bacterial. Preferably it is a viral vector, more preferably selected from adenoviral, adeno-associated viral, herpes, retroviral, lentiviral, RNA or DNA viral vectors. A preferred vector is lentiviral vector.

In a preferred embodiment, said vector comprises a oligonucleotide expressing or coding for a shRNA or said vector expresses a shRNA, said shRNA able to inhibit the expression of the FNIP2 gene. In a preferred embodiment, said vector comprises a nucleic acid coding for the shRNA, said nucleic acid which comprises or consists of one of the following sequences:
TL317378A (TL317378A) TCGGAGTTTGACCTGAATGAGATTCGCCT [SEQ ID N. 6]
TL317378B (TL317378B) CCAAGAAGGTCAACTGATGAGACATTCAG [SEQ ID N. 7]
TL317378C (TL317378C) GCTGTTGAAAGTGGAGATGCCTACAAGAC [SEQ ID N. 8]
TL317378D (TL317378D) AACGACCTGCCTCTGTTGACTGCTATTGC [SEQ ID N. 9]

Said polynucleotide can be administered by any administration route, for example by injection, preferably by intrathecal injection, but other routes are equally suitable.

Said polynucleotide can be included in a pharmaceutical composition further comprising at least one pharmaceutically acceptable vehicle and/or excipient. Suitable pharmaceutically acceptable excipients are those commonly known to the person skilled in the art for the preparation of compositions for parenteral, intradermal, subcutaneous, oral, transdermal, topical, transmucosal, and rectal administration.

By way of non-limiting example, said pharmaceutically acceptable excipients can consists of binders, diluents, lubricants, glidants, disintegrants, solubilizing (wetting) agents, stabilizers, colorants, anti-caking agents, emulsifiers, thickeners and gelling agents, coating agents, humectants, sequestrants, and sweeteners.

In a preferred embodiment, said pharmaceutical composition comprises a mixture of polynucleotides, said mixture preferably comprising two or more oligonucleotides having the sequences with SEQ ID N. 1, 2, 3, 4 and 5. Preferably, said composition comprising at least one oligonucleotide having a sequence with SEQ ID N. 1, at least one oligonucleotide having a sequence with SEQ ID N. 2, at least one oligonucleotide having a sequence with SEQ ID N. 3, at least one oligonucleotide having a sequence with SEQ ID N. 4 and at least one oligonucleotide having a sequence with SEQ ID N. 5.

Said pharmaceutical composition may further comprise one or more active pharmaceutical ingredients, such as one or more drugs or compounds which are useful in the treatment of ataxia telangiectasia, including, without limitation, acetylleucine, betamethasone, dexamethasone and Tempol (4-hydroxy-2,2,6,6-tetramethylpiperidin-1-oxyl).

According to the present invention, said polynucleotide may be administered to a subject affected by ataxia telangiectasia to treat the disease.

For treatment of ataxia telangiectasia is intended that administration of the inhibitor improves or treats the disease and/or one or more of its symptoms or the inhibitor blocks or slows down the progression of the disease.

Symptoms of ataxia telangiectasia which can be treated or improved by administration of a FNIP2 inhibitor comprise ataxia (i.e. difficulty with control of movement), oculomotor apraxia (i.e. difficulty with coordination of head and eye movement when shifting gaze from one place to the next), involuntary movements, telangiectasia (i.e. dilated blood vessels) over the white (sclera) of the eyes, problems with infections, especially of the ears, sinuses and lungs, increased incidence of cancer, slowed rate of growth (weight and/or height), drooling, dysarthria, diabetes.

In a particular embodiment, said polynucleotide is for use for the treatment and/or prevention of a disease associated with Ataxia Telangiectasia. Said disease can be selected from a cancer, a neurological disease, diabetes and liver steatosis.

Preferably, it is for use for the treatment or prevention of cancer in a subject affected by Ataxia Telangiectasia. Said cancer can be any cancer, preferably it is an hematological cancer such as lymphoma or leukaemia.

Preferably, it is for use for the treatment or prevention of a neurological disease in a subject affected by Ataxia Telangiectasia. Such neurological disease can be a neurodegenerative disease affecting cerebellar, cortical and/or spinal neurons.

It is also an object of the invention an inhibitor of protein FNIP2 for use in the treatment of Ataxia Telangiectasia.

The person skilled in the art is able to obtain an inhibitor of the FNIP2 protein as herein defined according to common knowledge in the field.

In particular, sequences of the FNIP2 gene and of the FNIP2 protein are known and the expert in the field may design molecules able to inhibit FNIP2 activity or expression.

Preferably, said inhibitor inhibits the expression of the gene coding for the FNIP2 protein, more preferably it is a polynucleotide as above defined.

A vector comprising or expressing for said inhibitor of protein FNIP2 or a pharmaceutical composition comprising said inhibitor of protein FNIP2 for use in the treatment of Ataxia Telangiectasia are also objects of the invention.

For "expressing an inhibitor of FNIP2" it is intended that the vector comprises a nucleic acid which when the vector is inserted in a cell is transcribed to a molecule able to inhibit the expression of the FNIP2 gene or able to inhibit the activity of the FNIP2 protein.

The invention will be now illustrated by the following examples.

### EXAMPLES

### Materials and methods

### Metabolite analysis

Metabolite extraction and Ultrahigh Performance Liquid Chromatography-Tandem Mass Spectroscopy analysis were performed by Metabolon (Durham, North Carolina) as previously described (Bruhn et al., 2020). Data analysis was performed in R (version 4.0.2) running in an RStudio environment (version 1.0.153). Missing metabolite intensities were imputed with the lowest detectable value, and intensities were median-centered. Significance of metabolite alterations were calculated with the maanova R package by pairwise comparison of indicated groups (version 1.58.0), and a FDR threshold of 0.1 was used to identify altered metabolites. Scatter plots and heat maps were generated with the R packages ggplot2 (version 3.3.3) and pheatmap (version 1.0.12), respectively. Principal component analysis calculation and visualization were done with the packages FactoMineR (version 2.4) and factoextra (version 1.0.7) with convex ellipse type. The sum of detectable oligosaccharides was calculated from maltose, maltotriose and maltotetraose.

### Glucose-derived carbons flux analysis.

Cells were exposed to [U-¹³C₆]-glucose 2.5 mM for 24 hours. Cells were smashed in a tissue lyser for 1 min at maximum speed in 250µl of ice-cold methanol/water 50:50. Lysates were spun at 15,000g for 15 min at 4°C and supernatants were then passed through a regenerated cellulose filter (4mm Ø, Sartorius). Samples were then dried under N2 flow at 40°C and resuspended in 100µl of ice-cold methanol for subsequent analyses.

Data were obtained by liquid chromatography coupled to tandem mass spectrometry. We used an API-3500 triple quadrupole mass spectrometer (AB Sciex, Framingham, MA, USA) coupled with an ExionLC^{™} AC System (AB Sciex). Quantification of isotopomers was performed by using a cyano-phase LUNA column (50mm x 4.6mm, 5µm; Phenomenex) by a 5 min run in negative ion mode. Mobile phase A was: water and phase B was: 2mM ammonium acetate in MeOH. Runs were performed in isocratic mode with 80% B with a flow rate of 500µl/min. Data are expressed as mass isotopomer distribution (MID).

### Cell Culture

Primary fibroblasts were obtained from the Coriell Institute. The cells were maintained in minimum essential medium (Sigma Aldrich; M2279) supplemented with 20% Fetal bovine serum (FBS) in humid incubator at 5% CO₂ and 3% O₂. The following cell lines were used for the experiments: CTRL: GM03492, GM03400, GM03396, AG03059, AG03057, AG02603, GM00024, AG02602; A-T: GM03487, GM03395, AG03058, GM05823, GM01588, GM00647, GM02530, GM00367, AG03058. HeLa cells were maintained in minimum essential medium (Sigma Aldrich; M2279) supplemented with 10% Fetal bovine serum (FBS) in humid incubator at 5% CO₂ and 20% O₂. hiPSC were reprogrammed from AG02602 (CTRL) and GM02530 (AT) primary fibroblasts using the Cytotune^{™}-iPS Sendai reprogramming kit (Invitrogen; A16517) and cultured on Matrigel (SACCO; 354277) coated plates in mTESR^{™1} (Stem Cell Technology, 85850) supplemented with ROCK inhibitor, Y27632 (ADOOQ; A11001) in the first 24 hours of culture. Neuronal differentiation was obtained according to manufacturer instruction (Stem Cell Technology, 08601)

### RNA extraction, cDNA synthesis and real time qPCR

Cells were washed with PBS and RNA was extracted using the RNeasy Mini Kit (QIAGEN, 74104) following manufacturer's instruction. RNA samples were purified from DNA contaminants by DNAse I digestion (QIAGEN, 79254). Yield and purity of RNA were assessed using spectrophotometer (Nanodrop, Thermofisher). cDNA samples were prepared using the iScript^{™} Reverse transcription Supermix kit (BioRad, 1708890) following the manufacturer's instructions and starting from 1 µg of RNA. Reverse transcription was performed in thermocycler according to the following program, 20 min at 42°C, 1 min at 95°C and ∞ at 4°C. The qPCR was performed based on standard protocol using a final working concentration of 1X SsoFast^{™} EvaGreen^{®} Supermix (Bio Rad, 1725201), 0.5 µM of primer mix and 5 ng of cDNA. Each sample was loaded three times for technical triplicates. The housekeeping gene, GAPDH, was used as loading control to normalize qPCR data following ΔΔCt method. Briefly, for each sample each gene was tested in technical triplicate using a specific pair of primers. The average threshold cycle (Ct) for each gene was then normalized for the average Ct calculated for GAPDH for the same sample obtaining a ΔCt. The ΔCt for each gene of each sample was then normalized again over the ΔCt for that gene on a control sample obtaining a ΔΔCt. The relative level of mRNA expression was then expressed as fold change (2^{-ΔΔCt}). The primers used are GAPDH Forward: CTTTGTCAAGCTCATTTCCTGG [SEQ ID N. 10] and Reverse: TCTTGCTCAGTGTCCTTGC [SEQ ID N. 11] while for FNIP2 the pre-designed KiCqstart Primer Pairs for human FNIP2 gene H_FNIP2_1 (Sigma Aldrich) was used.

### Transfection, esiRNA-mediated knock-down and LNA antisense

To knockdown our genes of interest, primary fibroblasts were transfected with endoribonuclease-prepared small interfering RNA (esiRNA), targeting the mRNA of a specific gene. The transfection reagent Lipofectamine RNAiMax (13778100, Invitrogen) was used, taking advantage of its low toxicity and high efficiency in the delivery of esiRNA into the cells. Once inside the cell, esiRNA induce RNA interference, leading to the degradation of mRNA and the knockdown of its resulting protein. Lipofectamine RNAiMax was diluted 1:20 in Opti-MEM Reduced serum Medium (Cat, ThermoFisher) and incubated at room temperature for 5 min. Simultaneously, esiRNA were diluted in Opti-MEM to reach a concentration of 60 nM to 150 nM, depending on previously optimized concentration. Lipofectamine and esiRNA were mixed 1:1 and incubated for 15 min at room temperature to allow complexes formation. Finally, the lipofectamine/esiRNA mixture was added dropwise to the seeded cells at 60-80% confluency and cells were incubated for 48 hrs to 72 hrs before sample collection to be assessed by qPCR, Western Blot (WB) or PAS staining. Similar transfection procedures were used for LNA antisense oligos. However, in this case a concentration of LNA varying between 80 and 200 nM was used. In all the experiments with esiRNA, mission esiRNA targeting luciferase (EHURLUC, Sigma Aldrich) was used as a negative control. The following esiRNA were used: mission esiRNA targeting human PYGL (EHU037491, Sigma Aldrich), mission esiRNA targeting human ATM (EHU089521, Sigma Aldrich), mission esiRNA targeting human FNIP2 (EHU054551, Sigma Aldrich), mission esiRNA targeting human FLCN (EHU006461, Sigma Aldrich) and ON-TARGETplus Human FNIP1 (96459) siRNA- SMARTpool (L-032573-02-0020, Horizon Discovery). Custom made LNA antisense oligos with the following sequences were also used. Asterisk indicates phosphorothioathe bond, + indicates LNAs.
LNA_1 FNIP2_1 LG00809562-DDA:
   +A*+G*+T*+A*G*G*T*C*A*G*G*A*+C*+A*+T*+A [SEQ ID N. 1]
LNA_2 FNIP2_2 LG00809563-DDA:
   +G*+A*+A*+A*G*T*A*G*G*T*C*A*+G*+G*+A*+C [SEQ ID N. 2]
LNA_3 FNIP2_3 LG00809564-DDA:
   +G*+T*+G*+G*A*G*G*A*G*A*A*C*+G*+T*+A*+T [SEQ ID N. 3]
LNA_4 FNIP2_4 LG00809565-DDA:
   +A*+C*+G*+G*C*T*G*A*C*A*T*A*+A*+A*+A*+C [SEQ ID N. 4]
LNA_5 FNIP2_5 LG00809566-DDA:
   +C*+G*+A*+T*T*C*T*G*A*G*G*A*+C*+A*+G*+C [SEQ ID N. 5]

### Protein extraction and Western blot

Cells were washed with PBS, trypsinized and pelleted by centrifugation. The pellet was resuspended and lysed in homemade RIPA buffer supplemented with protease inhibitor (Calbiochem). Samples where lysed at 4°C for 30 min, followed by sonication using BioRuptor Sonication system (UCD200, Diagenode) at high power for 6 cycles (30s on, 30s off) before centrifugation to pellet the cellular debris (15000 rpm, 30min, 4°C). Supernatants were transferred to clean Eppendorf tubes and protein concentration was assessed by Bradford assay measuring the absorbance at 595 nm. For SDS-PAGE, 20 µgs of proteins were mixed with Laemmli solution and placed for 10 min in a 95°C water-bath before being loaded on 4 - 15% Bis - Tris Poly Acrylamide gels (Biorad) soaked in homemade running buffer. Electrophoresis was performed at 120 V until the dye reached the bottom of the gel. Proteins were transferred from gel to 0.2 µm nitrocellulose membrane (Biorad) using the Trans-Blot Turbo Transfer system (Biorad). The filters were blocked for 1 hr on a rocker with 5% milk in TBST buffer (Tris pH 20 mM, NaCl 150 mM, 0.1% Tween). After blocking, the corresponding primary antibody was added and membranes were maintained at 4°C on rocker overnight, washed 3 times with TBST and incubated for at least 1 hr with the suitable secondary antibody conjugated to HRP (Horse radish peroxidase). Signals were detected using the ECL (GE-Healthcare). The signal was developed either by using the classical technique on photographic films in dark room, or using the Chemidoc machine (BioRad). The following antibodies were used at the concentration suggested by the manufacturer. ATM (SC-23921, Santa Cruz); PYGL (Ab223788, Abeam); FNIP2 (HPA042779); Atlas antibody); FNIP1 (Ab134969, Abcam); FLCN (3697s, Cell Signaling).

### PAS Staining and Microscopy

Periodic acid-Schiff (PAS) staining is a widely used technique to detect glycogen in tissues. The periodic acid oxidizes the vicinal diols in glycogen and creates free aldehydes groups that further react with Schiff reagent and produce a pink or magenta color. A positive PAS staining indicates the presence of glycogen in cells and tissues. Cells were grown on glass in 24-well plate or glass chamber (Cat). Samples were fixed with ethanol and 37% parafolmaldehye at ratio of 9:1 respectively, for 10 minutes and stained using the Periodic acid- Schiff kit (395B, MERCK) according to manufacturer's instruction. Briefly, fixed cells were treated with periodic acid solution for 5 minutes, washed with water and stained with Schiff reagent for 10 minutes then counterstained with hematoxylin provided with the kit for 90 s. The cells were dehydrated with increasing concentration of ethanol (70%, 85% and 100% respectively), air-dried and mounted with Eukitt medium. For cryosections, as in the case of tissues, samples were fixed with ethanol and chloroform at a ratio of 7:3 respectively, before being stained following the same protocol. To eliminate the possibility of detecting other polysaccharides than glycogen, a negative control treated with 0.5 % α-Amylase from porcrine pancreas (DIE) (A3176, Sigma Aldrich) for 5 min, was used for each condition prior to staining. The slides were analyzed by widefield microscopy using Olympus Upright BX51 Full Manual microscope. PAS staining intensity was quantified with Cell profiler image analysis software.

### Generation of Stable FNIP2 Knockdown with shRNA Vector

Four different constructs of FNIP2-human 29mer shRNA in lentiviral GFP vector (pGFP-C-shLenti, third generation) were obtained from Origene: TL317378A, TL317378B, TL317378C, TL317378D (sequences shown below) and one empty vector TR30021 for negative control. The dried plasmid was reconstituted in autoclaved MilliQ ddH₂O, diluted and added to thawed vials of the transformation competent Top10 E.coli strains for transformation followed by 30 min incubation on ice. The heat shock was performed at 42°C for 45 s and the vials were incubated for 2 min on ice. Antibiotics-free LB medium was added to the vials which were then placed in shaking incubator at 37°C, 225 rpm for 1 hr. Part of the samples were then spread on 34 µg/ml chloramphenicol plates and incubated at 37°C overnight for colony formation. The plasmid was then expanded and isolated using the Nucleobond Xtra Maxi kit (74041410) for low copy plasmid. The obtained plasmid was quantified and the size was verified using restriction enzyme digestion (Xba1, Xho1 and Bamh1). An agarose gel was run and the plasmid with the correct size (TL317378C) was used for infection.

The virus was produced in HEK293T cells according to the TR30037 packaging kit instructions. 293T cells were grown in in antibiotic-free medium and transfected with a mixture of packaging plasmid (TR30037), TL317378C (FNIP2 plasmid) or TR30021 (SCRAMBLE plasmid), and turbofectin in optimum-medium. The mixture was incubated for 15 min at room temperature and added dropwise to the 293T plates and incubated overnight. The next day medium was changed and cell were reincubated. For the next two days the medium was collected, filtered through a 0.45 µm filter to and stored at +4°C. Polyethylene-glycol (PEG) solution was added for precipitation of the virus. The virus was further resuspended in the corresponding fibroblasts medium and added to the attached fibroblasts in the presence of polybrene. Three days post infection, cells were selected with 3 µg/mL of puromycin and cells were further passaged and collected for knockdown assessment.
TL317378A (TL317378A) TCGGAGTTTGACCTGAATGAGATTCGCCT [SEQ ID N. 6]
TL317378B (TL317378B) CCAAGAAGGTCAACTGATGAGACATTCAG [SEQ ID N. 7]
TL317378C (TL317378C) GCTGTTGAAAGTGGAGATGCCTACAAGAC [SEQ ID N. 8]
TL317378D (TL317378D) AACGACCTGCCTCTGTTGACTGCTATTGC [SEQ ID N. 9]

### Mitochondrial Respiration and Glycolysis Assay

Mitochondrial respiration and glycolysis were assessed simultaneously in fibroblasts cells using the Seahorse XF extracellular flux analyzer XF24. Mitochondrial respiration was measure as oxygen consumption rate while glycolysis was measured as the extracellular acidification rate. For the oxygen consumption rate, 4 toxins were injected consecutively in the following order: 1µM of oligomycin for inhibition of ATP-synthase. 1 µM of carbonyl cyanide p-trifluoromethoxyphenylhydrazone (FCCP) as an uncoupler of oxidative phosphorylation, 1 µM of Rotenone as an inhibitor of complex I of the mitochondrial respiratory chain and 1 µM of antimycin, complex III inhibitor. The cells are plated at high confluency to prevent proliferation. Fibroblasts medium was replaced with Seahorse assay XF medium supplemented with 5 mM glucose, 1 mM pyruvate and 2 mM glutamine and the pH adjusted to 7. For ECAR analysis, the medium was supplemented with 2 mM glutamine and the following compounds were added: 30 mM glucose to activate glycolysis, 1 µM oligomycin to inhibit mitochondrial respiration and induce maximal glycolysis and 2-deoxyglucose to inhibit glycolysis (2DG).

### Crystal Violet Staining

Cells were plated in 48-well plate at 20,000 cells/well in triplicate in four plates for each day. At the corresponding time, medium was removed and cells were washed twice with PBS following fixation with 50% ethanol solution in PBS, a fixation method modified for virus infected cells, followed by fixation for 15 min with 100% ethanol solution at room temperature. Crystal violet solution (0.5%, 20% methanol) was added and cells were washed until washing solution was clear. Crystal violet was redissolved in glacial acetic acid solution and then transferred to 96-well plate to be read with spectrophotometry.

### β-Gal Staining

The staining was performed using the β-Gal Staining Kit (K1465-01, Invitrogen) according to the manufacturer's instructions.

### Tables

**Table1**

| Cells used for Set 1 metabolic analyses | | | | | |
|---|---|---|---|---|---|
| **SAMPLES** | **KINSHIP** | **AGE** | **SEX** | **CLINICAL STATUS** | **MUTATION** |
| C1 | unmatched | 35Y | F | Healthy-unknown | no |
| C2 | unmatched | 31Y | M | Healthy-unknown | no |
| C3 | unmatched | 35Y | F | Healthy-unknown | no |
| C4 | unmatched | 31Y | M | Healthy-unknown | no |
| P1 | proband | 13Y | M | AT patient | homozygous for 7913G>A |
| P2 | proband | 14Y | F | AT patient | unknown |
| P3 | proband | 13Y | M | AT patient | homozygous for 7913G>A |
| P4 | proband | 14Y | F | AT patient | unknown |
| P5 | proband | 14Y | F | AT patient | unknown |
| P6 | proband | 13Y | M | AT patient | homozygous for 7913G>A |

**Table2**

| Cells used for Set 2 metabolic analyses | | | | | | | |
|---|---|---|---|---|---|---|---|
| **SAMPLES** | **KINSHIP** | **AGE** | **SEX** | **CLINICAL STATUS** | **MUTATION** | **GENETIC OUTPUT** | **CLINICAL SYMPTONS** |
| C3 | A1 brother | 8Y | M | healthy-unknown | unknown | unknown | none |
| C4 | mother | 37Y | F | healthy-CARRIER | 8366A>T (exon 58) | truncation at codon 2756 | none |
| C5 | sister | 11Y | F | Healthy-unknown | unknown | unknown | none |
| C6 | brother | 17Y | M | healthy-unknown | unknown | unknown | more resistant to neocarzinostatin inhibition of DNA synthesis than normal fibroblasts |
| C7 | mother | 42Y | F | healthy-CARRIER | 7913G>A (exon 55) | termination at codon 2638 | none |
| C8 | A2 father | 48Y | M | Healthy-CARRIER | unknown | unknown | none |
| C9 | A2 mother | 47Y | F | Healthy-CARRIER | unknown | unknown | none |
| A1 | C3 brother C4 son | 7Y | M | AT patient | 8266A>T (exon 58)-mother 4bp ins pos 1141(ex11)-father | truncation at codon 2756 frameshift+truncation at codon 381 | neurological abnormalities sensitivity to X-rays |
| A2 | C8 son C9 son | 14Y | F | AT patient | unknown | unknown | A-T and acute leukemia |
| A3 | unmatched | 18Y | M | AT patient | unknown | complementation group D | increased X-ray induced sensitivity |
| A4 | unmatched | 15Y | F | AT patient | homozygous for 103C>T | stop codon pos. 35 (exon 5) | clinically affected |
| A5 | unmatched | 17Y | M | AT patient | unknown | unknown | neurologic disability and telangiectasia, elevated serum alpha-fetoprotein |
| A6 | unmatched | 8Y | M | AT patient | 19bp deletion pos 2251 88 bp deletion pos 6573 | frameshift and truncation at codon 750 27 aa in-frame deletion at codon 2191 | Decreased post-neocarzinostatin exposure colony forming ability |
| G6PD1 | unmatched | 12Y | M | Favism affected | 563C>T | Ser188Phe{5188F} | RBC deficient for G6PD(med type) |
| G6PD2 | unmatched | 16Y | M | Favism affected | 563C>T | Ser188Phe{S188F} | RBC deficient for G6PD(med type) |
| G6PD34 | unmatched | 14Y | M | Favism affected | 563C>T | Ser188Phe{S188F} | RBC deficient for G6PD(med type) |
| G6PD4 | unmatched | 8Y | M | Favism affected | 563C>T | Ser188Phe{S188F} | RBC deficient for G6PD(med type) |

### RESULTS

### Example 1

### Identification of metabolome alterations in A-T cells

To identify possible metabolic defects underlying A-T cells malfunction we performed untargeted profiling of cell metabolites in two large experimental sets in which we compared metabolic analyses performed on primary fibroblasts derived from AT patients and unaffected controls. To understand similarities and differences with conditions that might mimic AT at metabolic level, including increased oxidative stress, we included primary fibroblasts derived from patients affected by favism in which G6PD is defective and normal fibroblasts treated with an oxidative agent such as H₂O₂ chronically administered every 12 hours for 5 days (Figure 1A and Tables 1-2 ).

We extracted the water soluble and non-soluble metabolites using standard protocols and performed liquid (LC) and gas (GC) chromatography followed by mass spectrometry (MS) using standard metabolites as a reference. These experiments led to the identification of more than 250 metabolites in sets 1 and 2 (Figure 1A). We then analyzed sample similarities and comparability of the independent experiments by principal component analysis (PCA) considering 115 metabolites that could reliably be detected in both sets. In each set, control and AT samples were almost completely separated along two principal components, and control and AT samples from both independent sets were intersected, suggesting AT-specific, reproducible metabolite alterations (Figure 1B).

Since AT cells show markers of oxidative stress, we asked to which degree oxidative stress contributed to the overall AT signature. Significantly, PCA analysis of the metabolome datasets of control and AT cells with and without H₂O₂ treatment (set 1) showed that ATM deficiency and H₂O₂ separated samples along the same principal component (Figure 2A), suggesting similar metabolite alterations. Indeed, the metabolite fold-changes induced by either ATM mutation or H₂O₂ treatment correlated (R² = 0.57), and significance analysis performed with the R package maanova revealed that most of the AT signature was recapitulated by H₂O₂ treatment of control cells (Figure 2A). Notably, the effect of H₂O₂ on metabolite levels was more pronounced in control than in AT cells (Figure 2B and 2C), suggesting a saturation effect in AT cells. As expected, both ATM mutation and H₂O₂ strongly reduced the ratio of reduced (GSH) vs. oxidized glutathione (GSSG) (Figure 2D), which reflects the overall oxidative stress levels. The oxidation levels were higher in AT cells than in G6PD defective cells, suggesting that in contrast to AT G6PD defective cells were able compensate the absence of NADPH produced by G6PD with alternative pathways. The oxidated NAD⁺, which is reduced in AT cells, likely due to poly(ADP-ribose) polymerase hyper-activation, was similarly reduced by ATM mutation and chronic H₂O₂ treatment (Figure 1E).

Overall, these results indicate that oxidative stress dependent alterations recapitulating the ones present in A-T cells could be transiently induced in control cells. Such alterations were already presented in untreated AT cells suggesting that uncompensated oxidative stress is the major driver of metabolic alterations in AT cells.

### Example 2

### Defective oxidative PPP is not sufficient to recapitulate the AT metabolome

A major source of anti-oxidant NADPH, a powerful antioxidant molecule used by cells to face up oxidative damage and for lipid synthesis, is the PPP, which is also the major cellular producer of ribose-5-phosphate required for nucleic acid synthesis. We have previously shown that ATM stimulates G6PD, the limiting enzyme of the PPP. As defective G6PD may account or the oxidative stress-induced metabolome alterations we verified metabolites similarities and difference between AT and G6PD defective cells derived from patients affected by favism in which G6PD is defective due to partially inactivating mutations in G6PD gene. We therefore aligned the AT metabolome with the metabolite profiles of 4 G6PDH mutant cell lines, normalized with control cells. Surprisingly, defective G6PDH activity was not sufficient to cause AT-like metabolome alterations (Figure 3).

### Example 3

### AT glycolysis impairment revealed by flux analysis

To gain further insight on the metabolic defects of AT cells we performed metabolic flux analysis, which provides a dynamic picture of defined metabolic pathways. As the AT metabolic signature identified by the static metabolic analysis indicated issues with glucose metabolism, we monitored the glycolysis and tricarboxylic acids (TCA) cycle activities by supplementing uniformly ¹³C₆-labelled glucose (U-¹³C₆-glucose) to CTRL and AT cells in culture and quantified the percentage of labeled sugar metabolism intermediates at steady-state level. Strikingly, the analysis revealed AT cells showed reduced levels of M6 fructose-1,6-bisphosphate (Fru-1,6-BP), M3 lactate as well as M2 citrate and alpha-ketoglutarate and malate all of which are derived by a single round of glycolysis and TCA cycle. Also, the levels of M3 fumarate (three glucose-derived carbons), a hallmark of multiple rounds of glycolysis coupled to TCA cycle, were decreased in AT cells indicating that the flux of glucose-derived carbons is impaired in these cells compared to wild type. These results indicate profound defects in glucose usage through glycolysis and TCA in AT cells.

Intriguingly, by monitoring the incorporation of ¹³C-glucose in polysaccharides such as maltotetraose, we detected decreased labeled-maltotetraose accumulation in AT cells (Figure 4).

### Example 4

### AT glycolysis defects lead to widespread glycogen accumulation in AT cells and tissues

As ¹³C-labeled maltotetraose can be formed by glycogen synthesis starting from ¹³C labeled glucose followed by partial glycogenolysis, its accumulation might derive from partial glycogen synthesis and/or degradation. Enrichment of oligosaccarides in AT cells might reflect a global accumulation of glycogen due to ineffective usage of glucose. Accumulation of glucose-6-P due to ineffective glycolysis, PPP and TCA might be channeled into glycogen synthesis, the partial degradation of which might generate oligosaccharide intermediates such as maltose, maltotriose and maltotetraose (Figure 5A).

To verify this hypothesis, we monitored monitor glycogen levels in AT cells using PAS-amylase staining, which is the standard technique to detect glycogen. Strikingly, we found significant increase for of PAS positive material in AT patients derived primary fibroblasts cells (Figure 5B) compared to healthy controls. The staining was mostly diffuse in the cytoplasm with scattered clumps also present. Digital image analysis confirmed significant increase in PAS-stained material in at least two different primary AT cells (Figure 5C).

To test the relevance of these findings for the AT neuropathology we monitored the levels of glycogen accumulation in neurons derived from iPSCs and in brains from AT patients. Importantly, glycogen accumulation was also present in neurons differentiated from AT cells (Figure 5D). To confirm the cellular observations, we stained human brain sections using a glycogen specific antibody validated for immune histochemistry (IHC) staining. Using this tool, we were able to document high level of glycogen accumulation in several regions of human brain of AT patients but not in control brains. The most striking accumulation was seen in Purkinje cells, which in AT brain appeared to be dysmorphic with significant loss of the dendritic tree (Figure 5D).

### Example 5

### ATM activate glycolysis upon oxidative stress

To understand whether glycogen accumulation was directly linked to lack of ATM function and not due to proliferation arrest or senescence related processes we inactivated ATM by CRISPR/cas9 in proliferating HeLa cell lines isolating different ATM mutated clones. Significantly, lack of ATM resulted in acute increase of glycogen, indicating a primary role for ATM in preventing abnormal glycogen accumulation (Fig 6A, 6B and 6C).

As glucose uptake is compromised in AT cells one possible cause of glycogen accumulation is the inefficient usage of glucose. We have previously shown that ATM activates the PPP to counteract oxidative stress and DNA damage accumulation. However, link between ATM and glycolysis remains unclear. In particular it is unknown how glycolysis is regulated by ATM in the presence of stresses that activate ATM. To uncover these aspects of AT physiopathology we used Seahorse Extracellular Flux analyzer to quantify glycolysis in normal and AT cells under unchallenged conditions. As expected, glycolysis was activated following glucose supplementation in WT cells cultivated under normal conditions as shown by lactate production and consequent medium acidification over time (Figure 6D). However, glycolysis activation was significantly suppressed in AT cells (Figure 6D). Strikingly, oxidative stress induced by supplementation of H₂O₂, was able to stimulate glycolysis in WT but not in AT cells (Figure 6D). These results indicate that ATM is required to activate glycolysis following oxidative stress.

### Example 6

### A FNIP2-dependent checkpoint restraining AT cell glycolysis and proliferation

Inability of AT to process glucose might trigger a metabolic checkpoint, which affects cell growth and proliferation. A major regulator of cell metabolism is the FFF complex, members of which have been shown to inhibit glycolysis and restrain cell growth and proliferation. The mechanisms underlying the function of the FFF complex and its individual components are still poorly understood. As FFF components FNIP1, FNIP2 and FLCN play a major role in restraining energy metabolism individually and in the FFF complex we verified the effect of their downregulation on glucose processing using glycogen accumulation as marker of abnormal glucose metabolism. Significantly, we found that FNIP2 downregulation with validated esiRNA oligos suppressed glycogen accumulation in AT fibroblasts (Figures 7A).

To better understand the role of FNIP2 in AT cells we investigated the effects of its downregulation on AT primary fibroblasts proliferation and growth. AT fibroblasts grow poorly compared to WT cells and tend to enter premature senescence following 8-10 passages. At this stage primary AT fibroblasts slow down their proliferation rate, acquire flatted morphology, increased number of intracellular granules and tend to die spontaneously. We constructed lentiviruses encoding for shRNA against FNIP2 (shFNIP2) or unrelated control sequences (shSCR). shFNIP2 lentivirus effectively decreased FNIP2 protein (Figure 7B) expression levels compared to shSCR infected cells.

Strikingly, downregulation of FNIP2 restored normal cell growth and survival of AT primary fibroblasts (Figure 7C and 7D).

Consistent with this we found that LNA oligos that were able to downregulate FNIP2 mRNA levels (Figure 7E), when combined, improved survival of AT fibroblasts (Figure 7F) and iPSC-derived neurons (Figure7G).

These results suggest the existence of an FNIP2 dependent metabolic checkpoint that restrains AT cell growth, proliferation and survival linked to abnormal glucose metabolism, which can be overcome by inactivation of FNIP2.

### Example 7

### Suppression of FNIP2 restores glycolysis and mitochondrial respiration in AT defective cells

To understand the mechanisms leading to glycogen accumulation and impairment of AT cells growth regulated by FNIP2 we measured glucose and oxygen consumption rates in WT and AT cells infected with shFNIP2 or shSCR. To this end we used the Seahorse platform to measure basic, glucose stimulated and maximal glycolysis. As expected, mock treated AT cells showed decreased ability to metabolize glucose through glycolysis over time compared to mock treated CTRL cells. Remarkably, FNIP2 downregulation in AT cells showed a strong increase in basic, glucose stimulated and maximal glycolysis (Figure 8A-C). Downregulation of FNIP2 in WT cells did not increase glycolysis instead (Figure 8A-C). Overall, these results indicate that FNIP2 suppression specifically restore and stimulate glycolysis in AT cells.

We then measured oxygen consumption rates in similar conditions. As expected, mock treated AT cells hSCR infected cells showed decreased oxygen consumption rates and basal respiration (8D) compared to mock treated CTRL cells. Importantly, downregulation of FNIP2 induced a significant increase in oxygen consumption rates and basal respiration (8D) in AT but not in CTRL cells.

These results indicate that FNIP2 downregulation upregulates mitochondrial function specifically in AT cells. This effect is likely due to the release of the inhibition on mitochondrial function in AT cells exerted by high levels of FNIP2.

The effects on AT proliferation and growth improvement might therefore be ascribed to improved glycolysis and mitochondrial respiration in AT cells. Our results identify a novel FNIP2 dependent metabolic checkpoint preventing glycolysis and cell growth in the absence of ATM. These findings predict that downregulation of FNIP2 might be a valid strategy to restore normal glycolysis and cellular metabolism promoting primary AT patients' cell survival.

### Discussion

AT displays a complex phenotype. Among the major issues of AT there are cerebellar degeneration, ocular telangiectasia, immunodeficiency, premature aging and diabetes (Shiloh, 2014). These multiple organ failures are difficult to explain with a sole failure in DSBs response. The mechanisms leading to these phenotypes would be better explained by a more general metabolic impairment.

ATM protein belongs to the family of PI3 kinases, which control several aspects of energetic metabolism. ATM loss and malfunction have been implicated in mammalian models of metabolic disease, which is characterized by insulin resistance, increased cholesterol and lipid levels, high blood pressure, and atherosclerosis (Shoelson, 2006).

However, ATM role in controlling cellular metabolism has remained poorly understood so far.

Here, we have shown that ATM plays a major function in controlling glucose metabolism.

In particular, our results indicate that a) AT cells have profound impairment in glycolysis and mitochondrial respiration; b) impaired glycolysis leads to glycogen accumulation; c) AT cells are unable to efficiently control oxidative stress; d) uncontrolled chronic oxidative stress in AT cells underlines glucose usage impairment with consequent increased catabolism of alternative carbon sources, including amino acids; e) ATM is required for rapid mobilization of glucose and glycogen in response to oxidative stress; f) lack of ATM leads to upregulation of the FNIP2 protein; g) FNIP2 activates a metabolic checkpoint that suppresses glycolysis and mitochondrial respiration; h) suppression of FNIP2 protein expression restores glucose usage, and mitochondrial respiration rescuing primary AT cells growth and survival.

These findings indicate a major role for ATM in regulating the glucose usage in response to acute and chronic stresses elicited by oxidation of the cellular environment. Failure in this response could explain the many of the phenotypes associated to AT at cellular, tissue and organism level, which show similar metabolic impairments as documented by widespread glycogen accumulation in human primary cells, such fibroblasts and neurons and adult tissues such as muscle and brain. Importantly, failure to use glucose leads to increased catabolism of other carbon sources, likely mimicking a starvation condition. In the absence of ATM FNIP2 increased levels trigger a metabolic checkpoint that suppresses glycolysis and mitochondrial function. FNIP2 increase might be driven by increased ROS, which also lead to FNIP1 stabilization. FNIP2 stabilization shuts down mitochondrial function to recalibrate ROS accumulation. Inactivation of mitochondrial function might impair glycolysis by limiting pyruvate uptake. Downregulation of FNIP2 by reactivating mitochondria also induces glycolysis reactivation limiting glycogen accumulation although a direct role of FNIP2 in suppressing glycolysis cannot be excluded.

This defect might be contributed by limiting amount of NADPH, the production of which is impaired by the absence of ATM mediated stimulation of G6PD. This checkpoint might serve as surveillance mechanism activated in the presence of unresolved oxidative stress and metabolic impairment to prevent cell growth and proliferation when ROS levels is dangerously high especially in neurons.

The use of anti-sense oligo administered directly with an intrathecal injection to downregulate FNIP2 might represent a synthetic survival approach to restore optimal cellular function in AT patients.

### References

Ambrose, M., and Gatti, R.A. (2013). Pathogenesis of ataxia-telangiectasia: the next generation of ATM functions. Blood 121, 4036-4045.
Bruhn, C., Ajazi, A., Ferrari, E., Lanz, M.C., Batrin, R., Choudhary, R., Walvekar, A., Laxman, S., Longhese, M.P., Fabre, E., et al. (2020). The Rad53(CHK1/CHK2)-Spt21(NPAT) and Tel1(ATM) axes couple glucose tolerance to histone dosage and subtelomeric silencing. Nature communications 11, 4154.
Cosentino, C., Grieco, D., and Costanzo, V. (2011). ATM activates the pentose phosphate pathway promoting anti-oxidant defence and DNA repair. The EMBO journal 30, 546-555.
Hicks, J., Wartchow, E., and Mierau, G. (2011). Glycogen storage diseases: a brief review and update on clinical features, genetic abnormalities, pathologic features, and treatment. Ultrastruct Pathol 35, 183-196.
Matsuoka, S., Ballif, B.A., Smogorzewska, A., McDonald, E.R., 3rd, Hurov, K.E., Luo, J., Bakalarski, C.E., Zhao, Z., Solimini, N., Lerenthal, Y., et al. (2007). ATM and ATR substrate analysis reveals extensive protein networks responsive to DNA damage. Science 316, 1160-1166.
Russo, I., Cosentino, C., Del Giudice, E., Broccoletti, T., Amorosi, S., Cirillo, E., Aloj, G., Fusco, A., Costanzo, V., and Pignata, C. (2009). In ataxia-teleangiectasia betamethasone response is inversely correlated to cerebellar atrophy and directly to antioxidative capacity. European journal of neurology 16, 755-759.
Schalch, D.S., McFarlin, D.E., and Barlow, M.H. (1970). An unusual form of diabetes mellitus in ataxia telangiectasia. N Engl J Med 282, 1396-1402.
Shiloh, Y. (2014). ATM: Expanding roles as a chief guardian of genome stability. Experimental cell research 329, 154-161.
Shiloh, Y., Tabor, E., and Becker, Y. (1982). Colony-forming ability of ataxia-telangiectasia skin fibroblasts is an indicator of their early senescence and increased demand for growth factors. Experimental cell research 140, 191-199.
Shoelson, S.E. (2006). Banking on ATM as a new target in metabolic syndrome. Cell metabolism 4, 337-338.
Wingard, M.C., Frasier, C.R., Singh, M., and Singh, K. (2020). Heart failure and diabetes: role of ATM. Curr Opin Pharmacol 54, 27-35.

## Claims

1. A polynucleotide able to inhibit, at least partially, expression of the FNIP2 protein, wherein said polynucleotide is selected from a short hairpin RNA (shRNA), an oligonucleotide expressing or coding for a shRNA and an antisense oligonucleotide.

2. The polynucleotide according to claim 1 which is an antisense oligonucleotide, wherein at least one nucleotide is a chemically modified nucleotide and wherein said oligonucleotide comprises at least one phosphorothioathe bond between any nucleotides.

3. The polynucleotide according to claim 2, wherein said at least one chemically modified nucleotide is selected from a locked nucleic acid (LNA)-modified nucleotide, a 2'-O-methyl-modified nucleotide, a 2'-O-methoxyethyl modified nucleotide and a 20-[2-(N-Methylcarbamoyl)ethyl]- modified nucleotide, preferably it is a locked nucleic acid (LNA)-modified nucleotide.

4. The polynucleotide according to claim 2 or 3, wherein said antisense oligonucleotide comprises 4 to 6 LNAs on each end of the oligonucleotide.

5. The polynucleotide according to anyone of claims 2 to 4 which is an antisense oligonucleotide comprising or consisting of a sequence selected from the following sequences:
+A*+G*+T*+A*G*G*T*C*A*G*G*A*+C*+A*+T*+A [SEQ ID N. 1]
+G*+A*+A*+A*G*T*A*G*G*T*C*A*+G*+G*+A*+C [SEQ ID N. 2]
+G*+T*+G*+G*A*G*G*A*G*A*A*C*+G*+T*+A*+T [SEQ ID N. 3]
+A*+C*+G*+G*C*T*G*A*C*A*T*A*+A*+A*+A*+C [SEQ ID N. 4]
+C*+G*+A*+T*T*C*T*G*A*G*G*A*+C*+A*+G*+C [SEQ ID N. 5]
wherein the nucleotides preceded by a symbol "+" are LNAs and the symbol "*" indicates a phosphorothioathe bond between the nucleotides.

6. A vector comprising or expressing the polynucleotide of anyone of claims 1 to 5.

7. The vector according to claim 6, said vector comprising a nucleic acid coding for a shRNA, preferably said nucleic acid which comprises or consists of a sequence with SEQ ID N.6, 7, 8 or 9.

8. A pharmaceutical composition comprising at least one polynucleotide of anyone of claims 1 to 5 or a vector according to claim 6 or 7, said composition preferably comprising a mixture of oligonucleotides comprising or consisting of the sequences with SEQ ID N. 1, 2, 3, 4 and 5, and at least one pharmaceutically acceptable vehicle and/or excipient.

9. The polynucleotide according to anyone of claims 1-5 or the vector of claim 6 or 7 or the pharmaceutical composition of claim 8 for use as a medicament.

10. The polynucleotide according to anyone of claims 1-5 or the vector of claim 6 or 7 or the pharmaceutical composition of claim 8 for use in the treatment of Ataxia-Telangiectasia.

11. The polynucleotide of anyone of claims 1-5 or the vector of anyone of claims 6-7 or the pharmaceutical composition of claim 8 wherein said polynucleotide or said vector or said composition is for use for the treatment and/or prevention of a disease associated with ataxia telangiectasia, preferably said disease being selected from cancer, a neurological disease, diabetes and liver steatosis.

12. The polynucleotide of anyone of claims 1-5 or the vector of anyone of claims 6-7 or the pharmaceutical composition of claim 8 for use for the treatment and/or prevention of cancer and/or of a neurological disease in a subject affected by ataxia telangiectasia, said cancer being preferably an haematological cancer such as lymphoma or leukaemia.

13. An inhibitor of protein FNIP2 for use in the treatment of ataxia telangiectasia, preferably said inhibitor inhibits, at least partially, the expression of the gene coding for the FNIP2 protein.

14. The inhibitor for the use according to claim 13 which is selected from:
a) a polynucleotide;
b) a vector comprising or expressing said polynucleotide;
c) an host cell genetically engineered to express said polynucleotide;
d) a small molecule;
e) a peptide, a protein, an antibody, an antisense oligonucleotide, a siRNA, a shRNA, a locked nucleic acid (LNA) oligonucleotide, a recombinant virus, an antisense expression vector,
preferably it is the polynucleotide of anyone of claims 1 to 5.

15. A pharmaceutical composition comprising at least one inhibitor of FNIP2 protein and at least one pharmaceutically accepted vehicle and/or excipient or a vector comprising or expressing an inhibitor of the FNIP2 protein, in particular an inhibitor of the expression of the FNIP2 gene, for use in the treatment of ataxia telangiectasia.
